(19)

Europäisches
Patentamt
European
Patent Office
Office européen
des brevets

(11) **EP 3 851 521 A1**

(12) **EUROPEAN PATENT APPLICATION**
published in accordance with Art. 153(4) EPC

(43) Date of publication:
**21.07.2021 Bulletin 2021/29**

(21) Application number: **19858765.1**

(22) Date of filing: **09.09.2019**

(51) Int Cl.:
**C12N 5/0797** (2010.01)     **C12N 5/02** (2006.01)
**C12Q 1/06** (2006.01)

(86) International application number:
**PCT/JP2019/035318**

(87) International publication number:
**WO 2020/054647 (19.03.2020 Gazette 2020/12)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME
KH MA MD TN**

(30) Priority: **14.09.2018   JP 2018172407**

(71) Applicant: **Keio University
Tokyo, 108-8345 (JP)**

(72) Inventors:
• **MORIMOTO, Satoru
Tokyo 160-8582 (JP)**
• **ZHOU, Zhi
Tokyo 160-8582 (JP)**
• **OKANO, Hideyuki
Tokyo 160-8582 (JP)**

(74) Representative: **HGF
1 City Walk
Leeds LS11 9DX (GB)**

(54) **METHOD FOR PRODUCING ASTROCYTES**

(57) A method for producing astrocytes includes a step of dissociating an embryoid body into single cells and suspension-culturing the cells in a serum-free medium containing basic fibroblast growth factor (bFGF) and epidermal growth factor (EGF) to obtain a neural stem cell mass, and a step of dissociating the neural stem cell mass into single cells and adhesion-culturing the cells in a serum-free medium to obtain a cell population containing astrocytes.

EP 3 851 521 A1

**Description**

TECHNICAL FIELD

The present invention relates to a method for producing astrocytes. More specifically, the present invention relates to a method for producing astrocytes, astrocytes, a co-culture of astrocytes and neurons, a method for screening a therapeutic agent for a neurological disease, and a serum-free medium for inducing differentiation of neural stem cells into astrocytes. Priority is claimed on Japanese Patent Application No. 2018-172407, filed on September 14, 2018, the content of which is incorporated herein by reference.

BACKGROUND ART

A number of studies have reported the involvement of astrocytes in the pathophysiology of neurological diseases. In order to analyze the involvement of astrocytes in neurological diseases, it is necessary to prepare and analyze a large amount of astrocytes. Therefore, it is required to establish a technique for inducing differentiation of pluripotent stem cells or the like into astrocytes. For example, Patent Document 1 discloses a method for inducing astrocyte differentiation.

Citation List

Patent Document

[Patent Document 1]
Published Japanese Translation No. 2016-504014 of the PCT International Publication

DISCLOSURE OF INVENTION

Technical Problem

However, it is difficult to obtain a cell population of astrocytes having a purity close to 100% in a short period by conventional methods for inducing astrocyte differentiation. It is also considered that astrocytes are in a static state and acquire irreversible inflammation/reactivity in the presence of serum. However, it has not been possible to obtain mature astrocytes in a static state as conventional methods for inducing astrocyte differentiation use serum in the process of inducing differentiation.
Therefore, it is an object of the present invention to provide a technique for producing a cell population of mature astrocytes in a static state.

Solution to Problem

The present invention includes the following aspects:

[1] A method for producing astrocytes, including dissociating an embryoid body into single cells and suspension-culturing the cells in a serum-free medium containing basic fibroblast growth factor (bFGF) and epidermal growth factor (EGF) to obtain a neural stem cell mass, and dissociating the neural stem cell mass into single cells and adhesion-culturing the cells in a serum-free medium to obtain a cell population containing astrocytes.
[2] The method for producing astrocytes according to [1], in which the embryoid body is obtained by culturing pluripotent stem cells in a serum-free medium.
[3] The method for producing astrocytes according to [2], in which the pluripotent stem cells are induced pluripotent stem cells derived from a healthy person or induced pluripotent stem cells derived from a neurological disease patient.
[4] The method for producing astrocytes according to any one of [1] to [3], in which obtaining the neural stem cell mass includes dissociating the embryoid body into single cells and suspension-culturing the cells in a serum-free medium containing bFGF and EGF to obtain a primary neural stem cell mass, and dissociating the primary neural stem cell mass into single cells and suspension-culturing the cells in a serum-free medium containing bFGF and EGF to obtain a higher-order neural stem cell mass.

The method for producing astrocytes according to any one of [1] to [4], in which in obtaining the cell population containing the astrocytes, the serum-free medium further contains brain-derived neurotrophic factor (BDNF) and/or glial-cell derived neurotrophic factor (GDNF).

[6] The method for producing astrocytes according to any one of [1] to [5], in which a percentage of astrocytes in the cell population is 90% or more.

[7] The method for producing astrocytes according to any one of [1] to [6], in which the astrocytes are in a static state.

[8] A cell population contained in a container in a serum-free state, in which a percentage of astrocytes is 90% or more.

[9] The cell population according to [8], which is in a static state.

[10] The cell population according to [8] or [9], in which a cell density is $1 \times 10^5$ cells/mL or more.

[11] The cell population according to any one of [8] to [10], comprising 95% or more of glial fibrillary acidic protein (GFAP)-positive cells and 95% or more of S100 calcium binding protein B (S100B)-positive cells.

[12] The cell population according to any one of [8] to [11], in which when serum is added to the medium, an expression level of GFAP, NFIA, ALDH1L1 or EAAT2 increases.

[13] A co-culture of the cell population according to any one of [8] to [12] and neurons.

[14] A method for screening a therapeutic agent for a neurological disease, including co-culturing the cell population according to any one of [8] to [12] and neurons in the presence of a test substance, and evaluating neurite outgrowth, morphology, synaptic vesicles, gene expression, protein expression, or electrophysiological parameters of the neurons to obtain an evaluation result, in which a significant change in the evaluation result as compared to an evaluation result in the absence of the test substance indicates that the test substance is a candidate for a therapeutic agent for neurological diseases.

[15] A serum-free medium for inducing differentiation of neural stem cells into astrocytes, including transferrin, putrescine, insulin, progesterone, sodium selenite, and a B27 supplement.

[16] The serum-free medium for inducing differentiation of neural stem cells into astrocytes according to [15], further including BDNF and/or GDNF.


Advantageous Effects of Invention

According to the present invention, it is possible to provide a technique for producing a cell population of mature astrocytes in a static state.


BRIEF DESCRIPTION OF DRAWINGS

Fig. 1 is a schematic diagram representing a differentiation induction protocol used in Experimental Example 1.

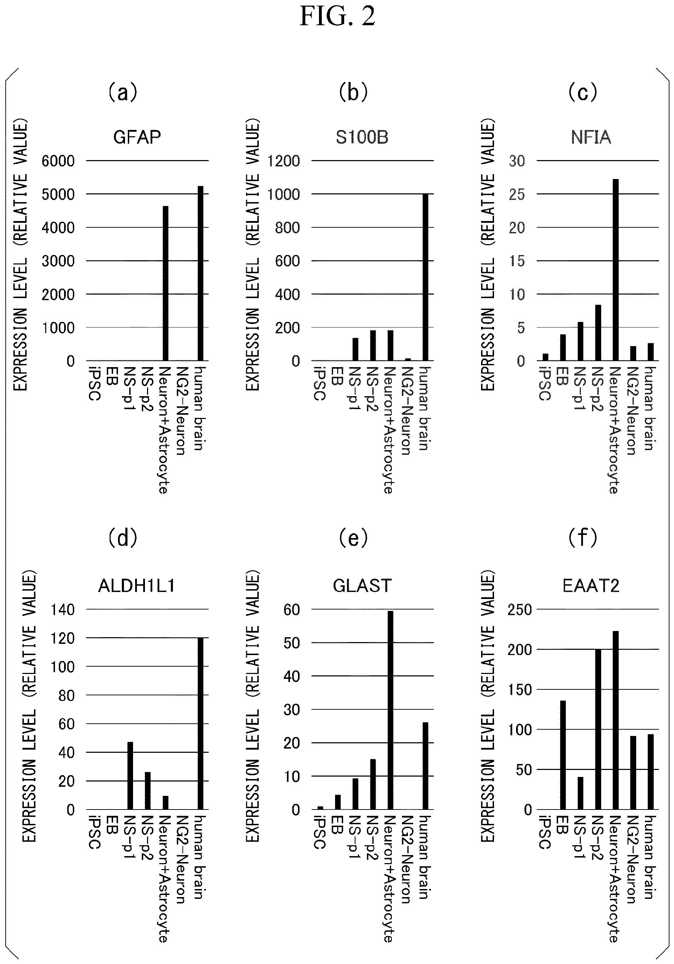

Figs. 2A to 2F are graphs showing the measurement results of expression levels of astrocyte-related genes in Experimental Example 2. Fig. 2A shows the measurement result of glial fibrillary acidic protein (GFAP), Fig. 2B shows the measurement result of S100 calcium binding protein B (S100B), Fig. 2C shows the measurement result of nuclear factor IA (NFIA), Fig. 2D shows the measurement result of aldehyde dehydrogenase 1 family member L1 (ALDH1L1), Fig. 2E shows the measurement result of glutamate aspartate transporter (GLAST, also called SLC1A3), and Fig. 2F shows the measurement result of excitatory amino acid transporter 2 (EAAT2, also called SLC1A2).

Figs. 3A to 3D are photographs showing the results of fluorescent immunostaining in Experimental Example 2. Fig. 3A is a photograph in which the expression of GFAP is detected, Fig. 3B is a photograph in which the expression of S100B is detected, Fig. 3C is a photograph in which the expression of NFIA is detected, and Fig. 3D is a photograph showing the result of staining the cell nucleus with Hoechst 33342.

Fig. 4 is a graph showing the results of measurement of changes over time in the concentration of glutamate in a cell medium in Experimental Example 3.

Fig. 5 is a fluorescence micrograph showing the detection of calcium ion influx into astrocytes in Experimental Example 3.

Figs. 6A to 6F are graphs showing the measurement results of expression levels of astrocyte-related genes in Experimental Example 4. Fig. 6A shows the measurement result of GFAP, Fig. 6B shows the measurement result of S100B, Fig. 6C shows the measurement result of NFIA, Fig. 6D shows the measurement result of ALDH1L1, Fig. 6E shows the measurement result of GLAST, and Fig. 6F shows the measurement result of EAAT2.

Figs. 7A to 7C are photographs showing the results of fluorescent immunostaining in Experimental Example 5. Fig. 7A is a photograph in which the expression of microtubule associated protein 2 (MAP2) is detected, Fig. 7B is a photograph in which the expression of Synapsin-1 is detected, and Fig. 7C is a photograph in which the expression of GFAP is detected, in which a photograph of cell nuclei stained with Hoechst 33342 is shown in the upper right frame of Fig. 7C.

Figs. 8A to 8D are fluorescence micrographs showing the results of Experimental Example 6.

Fig. 9 is a graph showing nerve spine density measured based on the results of Figs. 8A to 8D in Experimental Example 6.
Fig. 10 is a histogram of fluorescence intensity (relative value) measured for each astrocyte in Experimental Example 7.

DESCRIPTION OF EMBODIMENTS

[Method for producing astrocytes]

In one embodiment, the present invention provides a method for producing astrocytes, including dissociating an embryoid body into single cells and suspension-culturing the cells in a serum-free medium containing bFGF and EGF to obtain a neural stem cell mass, and dissociating the neural stem cell mass into single cells and adhesion-culturing the cells in a serum-free medium to obtain a cell population containing astrocytes.

As will be described later in Examples, the production method of the present embodiment can provide a technique for producing a cell population containing astrocytes at a high percentage. In addition, conventionally, it could take about half a year to induce the differentiation of astrocytes. On the other hand, according to the method of the present embodiment, astrocytes can be produced in about three months. Moreover, since the production method of the present embodiment performs all the steps in a serum-free state, astrocytes in a static state can be produced.

The astrocytes in a static state can be said to be astrocytes in an inactive state. As will be described later in Examples, whether or not the astrocytes are in a static state can be determined by, for example, the following method. First, serum is added to the astrocyte medium to be determined, and the expression levels of astrocyte-related genes such as GFAP, NFIA, ALDH1L1 and EAAT2 are measured before the addition of serum and after the addition of serum. As a result, when the expression level of the gene in the astrocytes after the addition of serum is higher than the expression level of the gene in the astrocytes before the addition of serum, it can be determined that the astrocytes are in a static state.

In the production method of the present embodiment, the embryoid body is preferably obtained by culturing pluripotent stem cells in a serum-free medium. This facilitates the production of astrocytes in a static state.

In the production method of the present embodiment, dissociating into a single cell means to dissociate the cells forming the cell mass one by one. Dissociation into a single cell can be performed by performing enzymatic treatment with trypsin, TrypLE Select, Accutase, or the like, which is usually used for cell dissociation, and pipetting.

Further, the embryoid body may be obtained from pluripotent stem cells cultured in a culture system that does not use feeder cells. By not using feeder cells, it is possible to produce an astrocyte cell population with further reduced impurity contamination.

The pluripotent stem cells described above may be, for example, ES cells, or may be, for example, induced pluripotent stem cells (iPSCs). Further, the pluripotent stem cells may be human-derived cells, or may be non-human animal-derived cells such as mice, rats, pigs, goats, sheep, and monkeys.

Further, the pluripotent stem cells described above may be induced pluripotent stem cells derived from a healthy person, or may be induced pluripotent stem cells derived from a neurological disease patient. When astrocytes are produced from induced pluripotent stem cells derived from a neurological disease patient, the obtained astrocytes can be used as a model for neurological diseases. Such astrocytes are useful for elucidating the mechanism of neurological diseases or the like.

In the production method of the present embodiment, the step of dissociating an embryoid body into single cells and suspension-culturing the cells to obtain a neural stem cell mass preferably includes a step of dissociating the embryoid body into single cells and suspension-culturing the cells in a serum-free medium containing bFGF and EGF to obtain a primary neural stem cell mass, and a step of dissociating the primary neural stem cell mass into single cells and suspension-culturing the cells in a serum-free medium containing bFGF and EGF to obtain a higher-order neural stem cell mass.

In the present specification, the neural stem cell mass obtained as a result of dissociating a primary neural stem cell mass into single cells and suspension-culturing the cells in a serum-free medium is referred to as a secondary neural stem cell mass. Further, the neural stem cell mass obtained as a result of dissociating the secondary neural stem cell mass into single cells and suspension-culturing the cells in a serum-free medium is referred to as a tertiary neural stem cell mass.

In the production method of the present embodiment, the dissociation into single cells and the formation of the neural stem cell mass are preferably repeated two or more times, and more preferably three or more times. As will be described later in Examples, by producing astrocytes via a higher-order neural stem cell mass, it is possible to produce an astrocyte cell population containing a high concentration of astrocytes exhibiting a static state.

The suspension culture of cells can be performed by culturing the cells in a culture vessel without a surface coating for cell adhesion.

In the production method of the present embodiment, in the step of dissociating the neural stem cell mass into single cells and adherently culturing the cells to obtain astrocytes, a serum-free medium may be used, a serum-free medium containing BDNF may be used, a serum-free medium containing GDNF may be used, or a serum-free medium containing BDNF and GDNF may be used.

Although BDNF and GDNF are not essential, by adding BDNF and/or GDNF to a serum-free medium, the resulting astrocyte morphology can be changed from an immature morphology such as radial glia to a more mature astrocyte morphology such as star morphology and amoeba morphology.

Further, as the serum-free medium, DMEM/F12 medium, MHM/B27 medium described later in Examples, or the like can be used.

The adhesion-culture of cells can be performed by culturing the cells using a culture vessel coated with a surface for cell adhesion. As the surface coating for cell adhesion, a laminin coat, a polyornithine coat, a poly-L-lysine coat, a Matrigel coat or the like may be used.

The percentage of astrocytes in the cell population obtained by the production method of the present embodiment is 90% or more, for example 95% or more, for example 100%. In the present specification, the percentage of astrocytes is 100%, which means that all the cells present in the culture vessel are astrocytes. Conventionally, it has been difficult to produce astrocytes of such purity. On the other hand, such astrocytes can be produced by the production method described above.

In the present specification, a high percentage of astrocytes in the cell population means a high percentage of astrocytes in the cells present in the culture vessel, and it can also be said that the differentiation efficiency of astrocytes is high.

In the production method of the present embodiment, when the neural stem cell mass is dissociated into single cells and is adhesion-cultured in a serum-free medium or in a serum-free medium containing BDNF and/or GDNF, astrocytes and neurons appear. At this stage, the percentage of astrocytes in the cell population is about 70%. When the astrocytes are removed from the culture vessel and passaged, the neurons disappear, and a cell population having a higher percentage of astrocytes can be obtained. After three passages, the percentage of astrocytes in the resulting cell population is approximately 100%. This passage can promote the differentiation of neural progenitor cells, remove emerging neurons, and lead immature astrocytes to maturity.

[Cell population]

In one embodiment, the present invention provides a cell population contained in a container in a serum-free state, in which the percentage of astrocytes is 90% or more, preferably 95% or more, and still more preferably 100%.

Conventionally, it has been difficult to produce a cell population containing astrocytes at such a percentage. On the other hand, such a cell population can be produced by the production method described above. The astrocytes in the cell population of the present embodiment are in a mature state, and can be conveniently used for research or the like immediately without waiting for a long preparation time. Conventionally, it could take about six months to produce astrocytes.

The astrocytes in the cell population of the present embodiment are preferably in a static state. It is possible to provide astrocytes while maintaining a static state by producing the astrocytes by the production method described above and storing the astrocytes in a container while maintaining a serum-free state.

Astrocytes in a static state are considered to be in a state closer to normal astrocytes present in vivo. Therefore, by performing analysis using astrocytes in a static state, it is possible to obtain experimental results that further reflect the state in vivo.

The cell population of the present embodiment may be contained in a container at a cell density of $1 \times 10^5$ cells/mL or more, for example, $5 \times 10^5$ cells/mL or more, for example $1 \times 10^6$ cells/mL or more. Conventionally, it has been difficult to produce such a large amount of astrocytes. On the other hand, such astrocytes can be produced by the production method described above.

The astrocytes of the present embodiment are in a frozen state, and may be contained in a cryotube or the like used for cryopreservation of normal cells. In this case, a commercially available cryopreservation solution can be used. Examples of the serum-free cryopreservation solution include CELLBANKER (registered trademark) 2 (Nippon Zenyaku Kogyo Co., Ltd.) and Bambanker (registered trademark) (LYMPHOTEC Inc.).

Further, as will be described later in Examples, the cell population according to the present embodiment is characterized in that the value $\alpha$ calculated by the following equation (1) with respect to the expression level of glutamate aspartate transporter (GLAST) and the expression level of GFAP is larger than the value $\alpha$ calculated by the following equation (1) with respect to the expression level of GLAST and the expression level of GFAP in human brain tissue.

$$\alpha = \text{GLAST expression level/GFAP expression level} \ldots (1)$$

Here, the value $\alpha$ may be calculated using total RNA derived from commercially available human brain tissue as the human brain tissue. The value $\alpha$ in the astrocytes of the present embodiment is, for example, 1.2 times the value $\alpha$ in human brain tissue, for example, 1.4 times, for example, 1.6 times, for example, 1.8 times, for example, 2 times.

Further, as will be described later in Examples, the cell population according to the present embodiment is characterized in that the value $\beta$ calculated by the following equation (2) with respect to the expression level of excitatory amino acid transporter 2 (EAAT2) and the expression level of GFAP is larger than the value $\beta$ calculated by the following equation (2) with respect to the expression level of EAAT2 and the expression level of GFAP in human brain tissue.

$$\beta = \text{EAAT2 expression level/GFAP expression level} ... (2)$$

Again, the value $\beta$ can be calculated using commercially available total RNA derived from human brain tissue as the human brain tissue. The value $\beta$ in the astrocyte of the present embodiment is, for example, 1.2 times the value $\beta$ in human brain tissue, for example, 1.4 times, for example, 1.6 times, for example, 1.8 times, for example, 2 times.

Further, as will be described later in Examples, the cell population of the present embodiment contains 95% or more, preferably 98% or more, and more preferably 100% of GFAP-positive cells. Further, the cell population of the present embodiment contains 95% or more, preferably 98% or more, and more preferably 100% of S100B-positive cells. Further, as will be described later in Examples, the cell population of the present embodiment contains 95% or more, preferably 98% or more, and more preferably 100% of NFIA-positive cells.

In addition, as will be described later in Examples, the cell population of the present embodiment is characterized in that the expression levels of GFAP, NFIA, ALDH1L1, EAAT2, and the like increase when serum is added to the medium.

Since the cell population of the present embodiment is produced by the production method described above, the astrocytes in the cell population are in a static state. Therefore, when serum is added to the medium, the expression levels of GFAP, NFIA, ALDH1L1, EAAT2, and the like are increased as compared with the expression levels in a serum-free state. For example, the degree of increase in the expression level of GFAP is, for example, 1.2 times, for example, 1.4 times, and for example, 1.6 times.

[Co-culture]

In one embodiment, the present invention provides a co-culture of the cell population described above and neurons.

Here, examples of the neurons include neurons induced to differentiate from pluripotent stem cells, and primary-cultured neurons. In this case, the pluripotent stem cells used for inducing differentiation of neurons may be the same as the pluripotent stem cells used for inducing differentiation of astrocytes. That is, the pluripotent stem cells described above may be, for example, ES cells, or may be, for example, induced pluripotent stem cells (iPSCs). Further, the pluripotent stem cells may be human-derived cells, or may be non-human animal-derived cells such as those from mice, rats, pigs, goats, sheep, and monkeys.

The cell population and co-culture of neurons described above can also be referred to as a co-culture system in which astrocytes and neurons are co-cultured. The co-culture is, that is, astrocytes and neurons co-cultured in a culture vessel, which may include a medium and a culture vessel.

By using the co-culture of the present embodiment, screening of therapeutic agents for neurological diseases, elucidation of mechanisms of neurological diseases, and the like can be performed.

[Method for screening a therapeutic agent for a neurological disease]

In one embodiment, the present invention provides a method for screening a therapeutic agent for a neurological disease, including a step of co-culturing the cell population described above and neurons in the presence of a test substance, and a step of evaluating neurite outgrowth, morphology, synaptic vesicles, gene expression, protein expression, or electrophysiological parameters of the neurons to obtain an evaluation result, in which a significant change in the evaluation result as compared to an evaluation result in the absence of the test substance indicates that the test substance is a candidate for a therapeutic agent for neurological diseases. According to the screening method of the present embodiment, a therapeutic agent for a neurological disease can be screened.

In the screening method of the present embodiment, the test substance is not particularly limited, and examples thereof include a natural compound library, a synthetic compound library, an existing drug library, and a metabolite library.

The evaluation of the neurite outgrowth of neurons may be performed by, for example, paraformaldehyde-fixing co-cultured astrocytes and neurons, immunostaining them with MAP2 or $\beta$3-tubulin and measuring the length of neurites by microscopic observation.

The evaluation of the morphology of neurons may be performed by, for example, paraformaldehyde-fixing co-cultured astrocytes and neurons, immunostaining them with MAP2 or $\beta$3-tubulin and evaluating them by microscopic observation, or evaluating the length and morphology of the neurites.

The evaluation of the synaptic vesicles of neurons may be performed by, for example, paraformaldehyde-fixing co-cultured astrocytes and neurons, immunostaining them with Synapsyn-1 and quantifying positive dots.

The evaluation of the gene expression of neurons may be performed by, for example, separating co-cultured astrocytes and neurons into respective cell types using a cell sorter or the like, and analyzing them by real-time quantitative PCR analysis, RNA-Seq analysis, or the like.

The evaluation of the protein expression in neurons may be performed by, for example, subjecting co-cultured astrocytes and neurons to immunostaining and Western blot analysis to analyze the expression of the protein of interest.

The evaluation of the electrophysiological parameters of neurons may be performed by, for example, a patch clamp method, calcium imaging method, or quantification of voltage-gated nerve activity using a microelectrode array.

When there is a significant change in the evaluation result of neurite outgrowth, morphology, synaptic vesicles, gene expression, protein expression, or electrophysiological parameters of the neurons in the presence of the test substance as compared to the evaluation result in the absence of the test substance, it indicates that the test substance is a candidate for a therapeutic agent for a neurological disease.

[Serum-free medium for inducing differentiation of neural stem cells into astrocytes]

In one embodiment, the present invention provides a serum-free medium for inducing differentiation of neural stem cells into astrocytes, including transferrin, putrescine, insulin, progesterone, sodium selenite, and a B27 supplement.

The neural stem cell is preferably a neural stem cell mass dissociated into single cells. Further, it is preferable that the medium of the present embodiment be used for adhesion-culture of neural stem cells.

Conventional media for inducing differentiation of neural stem cells into astrocytes contain serum. In contrast, the medium of the present embodiment is serum-free. Therefore, it is possible to induce differentiation of astrocytes in a static state.

Further, conventional media for inducing differentiation of neural stem cells into astrocytes contain platelet-derived growth factor (PDGF) and ciliary neurotrophic factor (CNTF), and are therefore costly. On the other hand, the medium of the present embodiment does not require these factors and is therefore inexpensive.

The medium of the present embodiment may further contain either BDNF or GDNF, or may further contain both BDNF and GDNF. Although BDNF and GDNF are not essential, by adding BDNF and/or GDNF to a serum-free medium, the resulting astrocyte morphology can be changed to a more mature astrocyte morphology such as star morphology and amoeba morphology.

EXAMPLES

Next, the present invention will be described in more detail with reference to Examples, but the present invention is not limited to the following Examples.

[Experimental Example 1]

(Induction of differentiation of human iPS cells into astrocytes)

Human iPS cells were induced to differentiate to produce astrocytes. As the human iPS cells, human iPS cell line 201B7 cells were used.

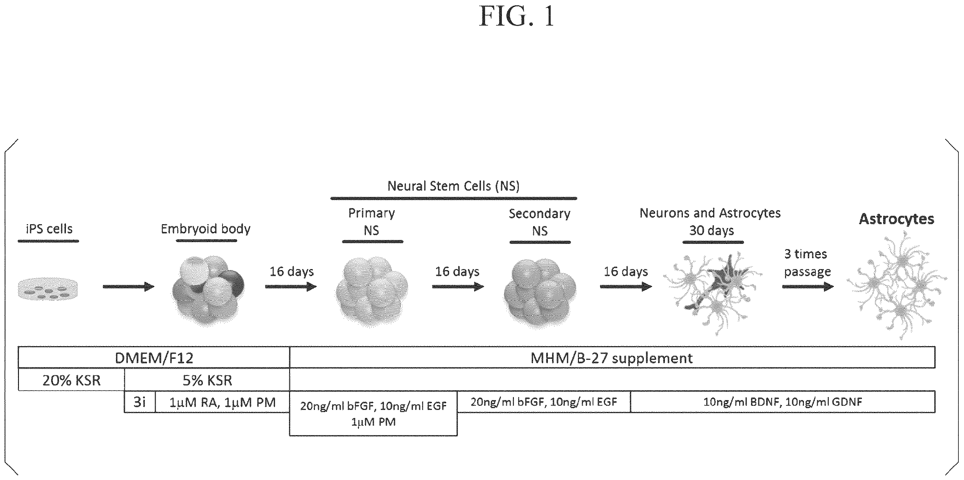

Fig. 1 is a schematic diagram representing a differentiation induction protocol used in this Experimental Example. In Fig. 1, "Embryoid body" means an embryoid body, "Neural Stem Cells (NS)" means a neural stem cell mass, "Primary NS" means a primary neural stem cell mass, "Secondary NS" means a secondary neural stem cell mass, "Neuron" means a neuron, and "Astrocyte" means an astrocyte. Further, "DMEM" means Dulbecco's Modified Eagle Medium, "F 12" means Ham's F12 Nutrient Mixture, "MHM" means media hormone mix medium, "KSR" means KnockOut (trademark) Serum Replacement, "3i" means 3 $\mu$M CHIR 99021, 3 $\mu$M SB 431542, 3 $\mu$M Dorsomorphine, "RA" means retinoic acid, "PM" means Purmorphamine, "bFGF" means basic fibroblast growth factor, also called FGF-2, "EGF" means epidermal growth factor, "BDNF" means brain-derived neurotrophic factor, and "GDNF" means glial cell line-derived neurotrophic factor.

The differentiation induction protocol shown in Fig. 1 does not consistently use serum. Hereinafter, a more detailed procedure for inducing differentiation will be described.

<< Human embryoid body (hEB) formation (day 0 to 15) >>

First, semi-confluent human iPS cells were prepared on a 10 cm dish containing a feeder. Subsequently, the feeder cells were peeled off with a dissociation solution T. The composition of the dissociation solution T is shown in Table 1 below. Subsequently, the cells were washed twice with 5 mL of PBS (-).

[Table 1]

| Dissociation solution T | |
| --- | --- |
| 2.5% trypsin | 5 mL |
| 1mg/mL collagenase IV | 5 mL |
| 1M CaCl$_2$ | 0.05 mL |
| KSR | 10 mL |
| Total | 50 mL |

Subsequently, 2 mL of hES medium was added per 10 cm dish. The composition of the hES medium is shown in Table 2 below.

[Table 2]

| hES medium | |
| --- | --- |
| DMEM/F12 | 500 mL |
| KSR (20%) | 125 mL |
| Nonessential amino acid (NEAA) | 5 mL |
| Diluted 2-mercaptoethanol | Final concentration 0.1mM |
| L-Glutamine | Final concentration 2mM |
| bFGF | Final concentration 5ng/mL |
| Penicillin/Streptomycin | Final concentration 0.5% |
| Total | 640 mL |

Subsequently, human iPS cell colonies were then removed from the dish using a scraper. Subsequently, the cells were collected in a 15 mL tube and allowed to stand at room temperature for 5 minutes. Subsequently, the supernatant was removed and 5 mL of bFGF-free hES medium was added. Subsequently, centrifugation was performed at 1,000 rpm at room temperature for 5 minutes.

Subsequently, 10 μM Y-27632 was placed in a 10 cm microbial dish. Subsequently, the supernatant after centrifugation was aspirated, 10 mL of bFGF-free hES medium was gently added, and the entire amount was transferred to a 10 cm microbial dish containing Y-27632. Subsequently, the cells were incubated overnight under the conditions of 37°C and 3% CO$_2$.

Subsequently, the next day, the medium was replaced with hEB medium (day 1). The composition of the hEB medium is shown in Table 3 below.

[Table 3]

| hEB medium | |
| --- | --- |
| DMEM/F12 | 500 mL |
| KSR (final concentration 5%) | 26 mL |
| Nonessential amino acid (NEAA) | 5 mL |
| Diluted 2-mercaptoethanol (final concentration 0.1mM) | 526 μL |
| L-Glutamine (final concentration 2mM) | 5 mL |
| Penicillin/streptomycin (final concentration 0.5%) | 5 mL |
| Total | 540 mL |

Subsequently, hEB was collected in a 15 mL tube and allowed to stand for 5 minutes. The medium was then aspirated and the cells were gently suspended in 10 mL of hEB medium containing 3 μM dorsomorphine (#P5499 Sigma), 3 μM SB 431542 (#S4317, Sigma) and 3 μM CHIR 99021 and returned to the microbial dish.

Subsequently, the medium was replaced with an hEB medium containing 1 μM retinoic acid (#R2625, Sigma) (day 4). Subsequently, the medium was replaced with an hEB medium containing 1 μM retinoic acid and 1 μM Purmorphamine (day 7). Thereafter, the medium was replaced with an hEB medium containing 1 μM retinoic acid and 1 μM Purmorphamine every 3 days (10th and 13th days).

<< EB separation (day 16) >>

Subsequently, the human embryoid body was collected in a 15 mL tube and allowed to stand for 5 minutes. Subsequently, the supernatant was removed, and the cells were suspended in 1 mL of TrypLE Select (Thermo Fisher Scientific), a cell dissociation enzyme. Subsequently, the cells were incubated in a warm bath at 37°C for 10 minutes. Subsequently, the cells were taken out of the warm bath, 1 mL of trypsin inhibitor was quickly added while observing the degree of cell separation, and pipetting was performed.

Subsequently, the human embryoid body was dissociated into single cells by pipetting 10 to 20 times with a 1000 μL pipette. Subsequently, the cells were passed through a cell strainer (70 μm, BD Falcon) to remove the remaining undissociated human embryoid body mass. Subsequently, 1 mL of MHM medium was poured onto the cell strainer and washed. Subsequently, centrifugation was performed at 1,000 rpm for 5 minutes. Subsequently, the supernatant was removed, 1 mL of MHM medium was added, and the number of cells was counted. The composition of the MHM medium is shown in Table 4 below.

[Table 4]

| Media Hormone Mix (MHM) medium | |
|---|---|
| 10×DMEM/F12 | 50 mL |
| 10×Hormone mix | 50 mL |
| 200mM L-Glutamine | 5 mL |
| Penicillin/streptomycin | (Final concentration 0.5%) 5 mL |
| 30% Glucose | 10 mL |
| 7.5% NaHCO$_3$ | 7.5 mL |
| 1M HEPES | 2.5 mL |
| Water | 375 mL |
| Total | 500 mL |

| 10×Hormone Mix | |
|---|---|
| Water | 652 mL |
| 10×DMEM/F 12 | 80 mL |
| 30% Glucose | 16 mL |
| 7.5% NaHCO$_3$ | 12 mL |
| 1M HEPES | 4 mL |
| Transferrin | 800mg |
| Putrescine | 77mg |
| Insulin | 200mg |
| 2 mM progesterone | 80μL |
| 3 mM sodium selenite | 80μL |
| Total | 764 mL |

In a T75 flask, 30 mL of proliferation medium containing 20 ng/mL of FGF (#100-18B, Peprotech), 10 ng/mL of EGF (#AF-100-15", Peprotech), and 1 $\mu$M Purmorphamine was prepared, and the cells were seeded at $1 \times 10^5$ to $4 \times 10^5$ cells/mL and placed in an incubator at 37°C and 5% $CO_2$. The composition of the proliferation medium is shown in Table 5 below.

[Table 5]

| proliferation medium | |
|---|---|
| MHM | 500 mL |
| B27 (containing Vitamin A) | (Final concentration 2%)10 mL |
| NEAA | 5 mL |
| Total | 515 mL |

<< Maintenance of primary neural stem cell mass (days 17 to 31) >>

To a T75 flask, 20 ng/mL of FGF and 10 ng/mL of EGF were added (day 20). Subsequently, the primary neural stem cell mass was collected in a 50 mL tube and centrifuged at 1,000 rpm for 10 minutes. In a T75 flask, 30 ml of new proliferation medium containing 20 ng/mL of FGF, 10 ng/mL of EGF, and 1 $\mu$M Purmorphamine was prepared, and all cells were transferred to the flask to replace the medium (day 24). Subsequently, 20 ng/mL of FGF and 10 ng/mL of EGF were added to a T75 flask (day 28).

<< Passage of primary neural stem cell mass (day 32) >>

The primary neural stem cell mass was collected in a 50 mL tube and centrifuged at 1,000 rpm for 5 minutes. Subsequently, the supernatant was removed, and the primary neural stem cell mass was suspended in 1 mL of TrypLE Select. Subsequently, the cells were incubated in a warm bath at 37°C for 10 to 15 minutes. Subsequently, 1 mL of trypsin inhibitor was quickly added and mixed while observing the degree of cell separation.

Subsequently, pipetting was performed 20 to 30 times with a 1000 $\mu$L pipette to dissociate the primary neural stem cell mass into single cells. Subsequently, the cells were passed through a cell strainer (70 $\mu$m, BD Falcon) to remove the remaining undissociated primary neural stem cell mass. Subsequently, 1 mL of MHM/B27 was poured onto the cell strainer for washing. Subsequently, centrifugation was performed at 1,000 rpm for 5 minutes. Subsequently, the supernatant was removed, 1 mL of MHM/B27 was added, and the number of cells was counted.

Subsequently, 30 mL of proliferation medium containing 20 ng/mL of FGF and 10 ng/mL of EGF was prepared in a T75 flask and the cells were seeded at $1 \times 10^5$ to $4 \times 10^5$ cells/mL and placed in an incubator at 37°C and 5% $CO_2$.

<< Maintenance of secondary neural stem cell mass (days 33 to 48) >>

To a T75 flask, 20 ng/mL of FGF and 10 ng/mL of EGF were added (day 36). Subsequently, the secondary neural stem cell mass was collected in a 50 mL tube and centrifuged at 1,000 rpm for 10 minutes. In a T75 flask, 30 ml of new proliferation medium containing 20 ng/mL of FGF and 10 ng/mL of EGF was prepared, and all cells were transferred to the flask to replace the medium (day 40). Subsequently, 20 ng/mL of FGF and 10 ng/mL of EGF were added to a T75 flask (day 44).

<< Terminal differentiation into astrocytes (day 48 to 76) >>

The secondary neural stem cell mass was collected in a 50 mL tube and centrifuged at 1,000 rpm for 5 minutes. Subsequently, the supernatant was removed, and the secondary neural stem cell mass was suspended in 1 mL of TrypLE Select. Subsequently, the cells were incubated in a warm bath at 37°C for 10 to 15 minutes. Subsequently, 1 mL of trypsin inhibitor was quickly added and mixed while observing the degree of cell separation.

Subsequently, pipetting was performed 20 to 30 times with a 1000 $\mu$L pipette to dissociate the secondary neural stem cell mass into single cells. Subsequently, the cells were passed through a cell strainer (70 $\mu$m, BD Falcon) to remove the remaining undissociated secondary neural stem cell mass. Subsequently, 1 mL of MHM/B27 was poured onto the cell strainer for washing. Subsequently, centrifugation was performed at 1,000 rpm for 5 minutes. Subsequently, the supernatant was removed, 1 mL of MHM/B27 was added, and the number of cells was counted.

Subsequently, the cells were suspended in differentiation medium and seeded at $4 \times 10^5$ cells/well on a 6-well plate coated for 1 hour with Matrigel diluted 200 fold with PBS (-). The composition of the differentiation medium is shown in Table 6 below.

[Table 6]

| differentiation medium | |
| --- | --- |
| Proliferation medium | remainder |
| rhBDNF (human recombinant BDNF) (#248-BD, R&D systems) | final concentration 10 ng/mL |
| rhGDNF (human recombinant GDNF) (#450-10, Peprotech) | final concentration 10 ng/mL |

Subsequently, the cells were placed in an incubator at 37°C and 5% $CO_2$. Subsequently, the medium was replaced every 7 days using 2 mL of differentiation medium (the cells were designated as A0).

<< Purification of astrocytes (days 77 to 98) >>

Subsequently, the cells were passaged from A0 to A1. First, the medium was removed, 700 μL/well of a cell exfoliating solution (trade name "Accutase", Funakoshi Co., Ltd.) was added, and the cells were incubated at 37°C for 30 to 60 minutes.

Subsequently, using a 1000 μL pipette, cells in each well of the 6-well plate were pipetted off and collected in a 15 mL tube. Subsequently, all wells of the 6-well plate were washed sequentially with 1 mL of differentiation medium and the medium was collected in the same 15 mL tube (7 mL in total).

Subsequently, centrifugation was performed at 1,000 rpm for 5 minutes. Subsequently, the supernatant was removed, 6 mL of differentiation medium was added, and strong pipetting was performed 20 to 30 times.

Subsequently, 1 mL/well of differentiation medium was added to a 6-well plate coated with Matrigel for 1 hour. Subsequently, a cell suspension with 6 mL of differentiation medium was seeded 1 mL at a time into each well of the 6-well plate. Subsequently, the cells were placed in an incubator at 37°C and 5% $CO_2$. Subsequently, in the same manner as the passage from A0 to A1, passage from A1 to A2 was performed on day 91, and passage from A2 to A3 was further performed on day 98.

By the method as described above, astrocytes could be induced to differentiate from human iPS cells. It is considered that astrocytes acquire irreversible inflammation/reactivity in the presence of serum. On the other hand, since the above method is performed without serum, mature astrocytes in a static state can be obtained by the above method. Further, the astrocytes induced to differentiate by the above method could be cryopreserved in a serum-free state at the stage of mature astrocytes and thawed. Further, the astrocytes could be induced in the same manner from iPS cells in a culture system without using feeder cells.

[Experimental Example 2]

(Examination of gene expression of astrocytes induced to differentiate from human iPS cells)

The expression of astrocyte-related genes at each differentiation stage of astrocytes induced to differentiate in the same manner as in Experimental Example 1 was examined by a quantitative RT-PCR method. Glial fibrillary acidic protein (GFAP), S100 calcium binding protein B (S100B), nuclear factor IA (NFIA), aldehyde dehydrogenase 1 family member L1 (ALDH1L1), glutamate aspartate transporter (GLAST, also called SLC1A3), and excitatory amino acid transporter 2 (EAAT2, also called SLC1A2) were examined as astrocyte-related genes.

Figs. 2A to 2F are graphs showing the measurement results of the expression level of each gene. Fig. 2A is a graph showing the measurement result of GFAP, Fig. 2B is a graph showing the measurement result of S100B, and Fig. 2C is a graph showing the measurement result of NFIA, Fig. 2D is a graph showing the measurement result of ALDH1L1, Fig. 2E is a graph showing the measurement result of GLAST, and Fig. 2F is a graph showing the measurement result of EAAT2. In Figs. 2A to 2F, the vertical axis indicates the relative value of the expression level, "iPSC" means iPS cells before the induction of differentiation into astrocytes, "EB" means embryoid body in the process of the induction of differentiation into astrocytes, "NS-p1" means primary neural stem cell mass, "NS-p2" means secondary neural stem cell mass, and "Neuron + Astrocyte" means cells on day 30 after the start of induction of differentiation in Experimental Example 1. Astrocytes at this stage contained neurons of about 70% percentage. Further, "NG2-Neuron" means pure neurons prepared by introducing Neurogenin-2 gene into iPS cells, and "human brain" means human brain tissue. As "human brain", total RNA derived from human brain tissue (#639320, Clontech) was used.

As a result, it was found that the expression level of the astrocyte-related gene tended to increase as the induction of differentiation of iPS cells into astrocytes progressed.

Further, the astrocytes induced to differentiate from iPS cells in the same manner as in Experimental Example 1 were characterized in that the value α calculated by the following equation (1) with respect to the expression levels of GLAST and GFAP was larger than the value α calculated by the following equation (1) for the expression levels of GLAST and GFAP in human brain tissue.

$$\alpha = GLAST\ expression\ level/GFAP\ expression\ level\ ...\ (1)$$

That is, in the astrocytes induced to differentiate from iPS cells in the same manner as in Experimental Example 1, the expression level of GFAP is about the same as the expression level of GFAP in human brain tissue, and the expression level of GLAST was about twice the expression level of GLAST in human brain tissue.

Further, the astrocytes induced to differentiate from iPS cells in the same manner as in Experimental Example 1 were characterized in that the value β calculated by the following equation (2) with respect to the expression levels of EAAT2 and GFAP was larger than the value α calculated by the following equation (2) for the expression levels of EAAT2 and GFAP in human brain tissue.

$$\beta = EAAT2\ expression\ level/GFAP\ expression\ level\ ...\ (2)$$

That is, in the astrocytes induced to differentiate from iPS cells in the same manner as in Experimental Example 1, the expression level of GFAP was about the same as the expression level of GFAP in human brain tissue, and the expression level of EAAT2 was about twice the expression level of EAAT2 in human brain tissue.

Subsequently, the expression levels of GFAP, S100B, and NFIA in astrocytes on day 51 after the start of induction of differentiation were examined by fluorescent immunostaining in the same manner as in Experimental Example 1.

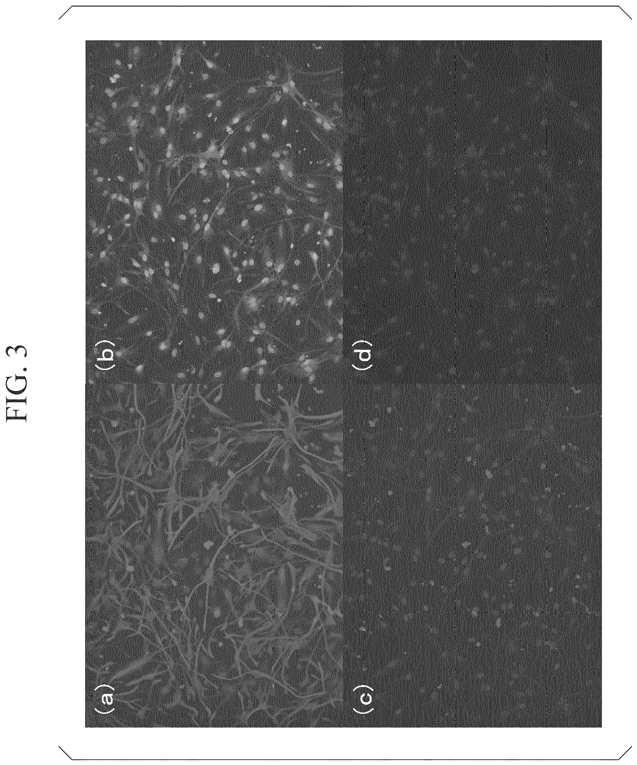

Figs. 3A to 3D are photographs showing the results of fluorescent immunostaining. Fig. 3A is a photograph in which the expression of GFAP was detected, Fig. 3B is a photograph in which the expression of S100B was detected, Fig. 3C is a photograph in which the expression of NFIA was detected, and Fig. 3D is a photograph showing the result of staining the cell nucleus with Hoechst 33342.

As a result, it was confirmed that the cells induced to differentiate in the same manner as in Experimental Example 1 had the shape of astrocytes. It was also revealed that all Hoechst 33342-positive cells expressed GFAP, S100B, and NFIA. This result means that almost 100% of the cells obtained by inducing differentiation were astrocytes.

[Experimental Example 3]

(Functional analysis 1 of astrocytes induced to differentiate from human iPS cells)

The function of astrocytes induced to differentiate was analyzed in the same manner as in Experimental Example 1. Specifically, the glutamate uptake and responsiveness to glutamine receptor stimulation in astrocytes on day 51 after the start of induction of differentiation were examined in the same manner as in Experimental Example 1.

<< Glutamate uptake >>

Astrocytes are known to take up glutamate. Therefore, the glutamate concentration in the medium after the addition of glutamate was measured over time for each of the astrocytes induced to differentiate in the same manner as in Experimental Example 1 and the human iPS cell line 201B7 used for the induction of differentiation.

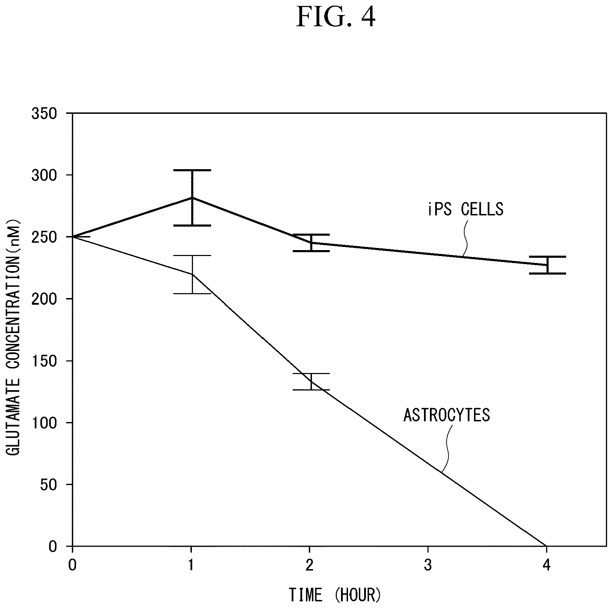

Fig. 4 is a graph showing the results of measuring the glutamate concentration in the medium in each cell. As a result, it was confirmed that the astrocytes induced to differentiate in the same manner as in Experimental Example 1 take up glutamate in the medium. This result indicates that the astrocytes induced to differentiate in the same manner as in Experimental Example 1 are functional astrocytes.

<< Responsiveness to glutamate receptor stimulation >>

Astrocytes induced to differentiate in the same manner as in Experimental Example 1 were added to the medium with Fluo-4, a calcium fluorescent indicator, at a final concentration of 5 μg/mL, and incubated at 37°C for 30 minutes to 1 hour. Subsequently, time-lapse photography was performed every 10 seconds using a fluorescence microscope.

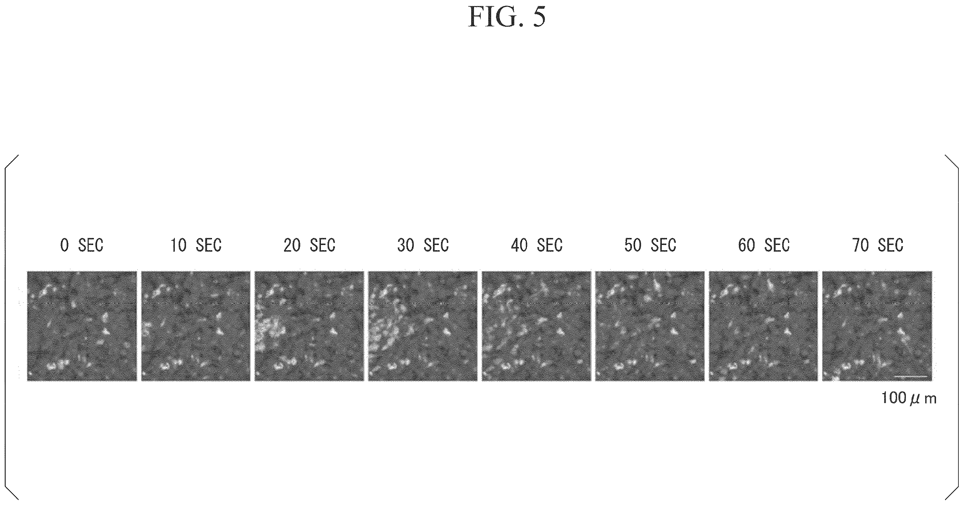

Fig. 5 shows a fluorescence micrograph showing the detection of calcium ion influx into astrocytes induced to differentiate in the same manner as in Experimental Example 1. The scale bar is 100 μm. As a result, it was observed that the signal indicating the influx of calcium ions was propagated to the adjacent astrocytes over time. This result further supports that the astrocytes induced to differentiate in the same manner as in Experimental Example 1 are functional astrocytes.

[Experimental Example 4]

(Effect of serum addition of astrocytes induced to differentiate from human iPS cells to medium)

The astrocytes induced to differentiate in the same manner as in Experimental Example 1 are in a serum-free state and in a static state. Serum was added to the culture medium of the astrocytes, and changes in the expression levels of astrocyte-related genes were examined by quantitative RT-PCR. As astrocyte-related genes, the same genes as in Experimental Example 2, that is, GFAP, S100B, NFIA, ALDH1L1, SLC1A3, and SLC1A2 were examined.

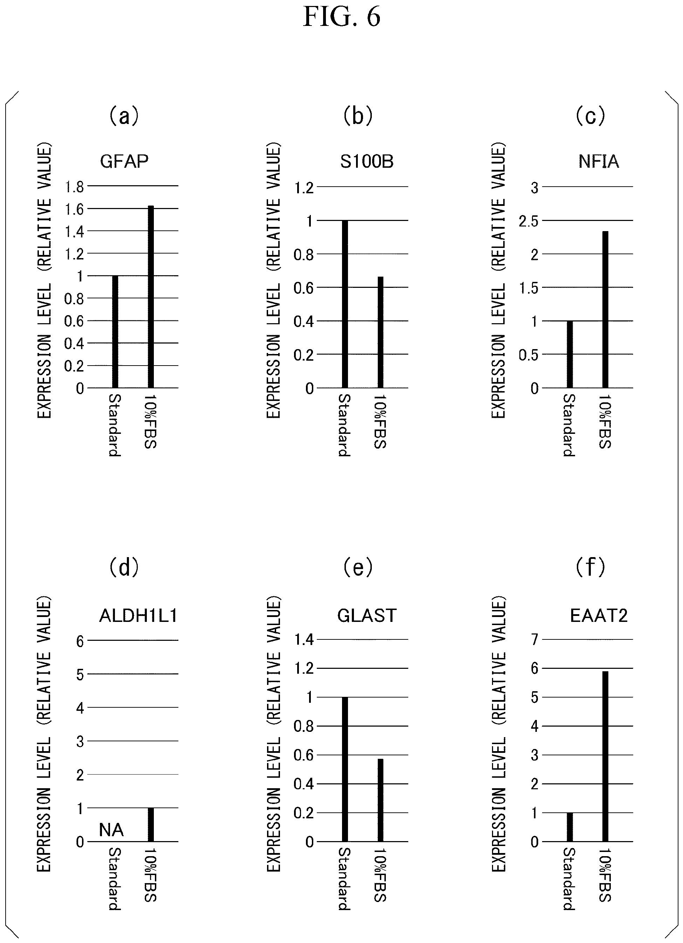

Figs. 6A to 6F are graphs showing the measurement results of the expression level of each gene. Fig. 6A is a graph showing the measurement result of GFAP, Fig. 6B is a graph showing the measurement result of S100B, and Fig. 6C is a graph showing the measurement result of NFIA, Fig. 6D is a graph showing the measurement result of ALDH1L1, Fig. 6E is a graph showing the measurement result of GLAST, and Fig. 6F is a graph showing the measurement result of EAAT2. In Figs. 6A to 6F, the vertical axis indicates the relative value of the expression level, "standard" means astrocytes in a serum-free state, and "10% FBS" means astrocytes supplemented with 10% fetal bovine serum (FBS) in the medium.

As a result, it was clarified that the expression levels of GFAP, NFIA, ALDH1L1 and EAAT2 increased when serum was added to the medium for the astrocytes induced to differentiate in the same manner as in Experimental Example 1. This result indicates that the addition of serum to the medium induces the activation of astrocytes induced to differentiate in the same manner as in Experimental Example 1.

[Experimental Example 5]

(Co-culture 1 of astrocytes induced to differentiate from human iPS cells and neurons)

The astrocytes induced to differentiate in the same manner as in Experimental Example 1 and neurons were co-cultured. As the neurons, neurons induced to differentiate from iPS cells were used.

First, neurons were induced to differentiate from iPS cells. Subsequently, astrocytes induced to differentiate in the same manner as in Experimental Example 1 were seeded in the culture medium of the neurons, and were co-cultured for 30 days.

Cells were then paraformaldehyde-fixed and fluorescently immunostained for neuronal markers microtubule associated protein 2 (MAP2) and Synapsin-1 and astrocyte marker GFAP.

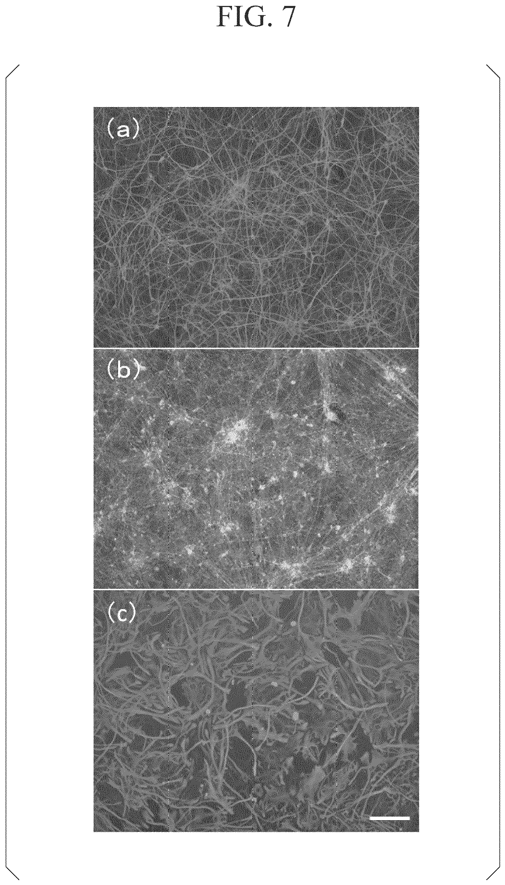

Figs. 7A to 7C are photographs showing the results of fluorescent immunostaining. Fig. 7A is a photograph in which the expression of MAP2 was detected, Fig. 7B is a photograph in which the expression of Synapsin-1 was detected, and Fig. 7C is a photograph in which the expression of GFAP was detected. The scale bar is 100 $\mu$m.

As a result, neurons and astrocytes were detected. Further, although astrocytes were added to the neuron culture system later, they remained under the neurons. This condition is similar to the arrangement of astrocytes and neurons in vivo.

By using this co-culture system, it is possible to analyze neurite outgrowth, morphology, synaptic vesicles, gene expression, protein expression, electrophysiological changes in synaptic responses, and the like of the neurons. In addition, by analyzing these changes in the presence of the test substance, it is possible to screen a therapeutic agent for a neurological disease. Further, the mechanism of disease can be analyzed by analyzing the co-culture system using neurons induced to differentiate from iPS cells derived from a disease patient and astrocytes induced to differentiate from iPS cells derived from a disease patient.

[Experimental Example 6]

(Co-culture 2 of astrocytes induced to differentiate from human iPS cells and neurons)

Apolipoprotein E is one of the major apolipoproteins that make up lipoproteins such as VLDL, IDL, and HDL. The APOE gene encoding apolipoprotein E has three alleles, $\varepsilon2$, $\varepsilon3$, and $\varepsilon4$, and apolipoprotein E has three isoforms, E2, E3, and E4, corresponding to each allele. The apolipoprotein E3 encoded by $\varepsilon3/\varepsilon3$ allele is a normal type (wild type), and the apolipoprotein E4 encoded by $\varepsilon4/\varepsilon4$ allele is considered to be a risk factor for Alzheimer's disease (AD). Further, the apolipoprotein E2 encoded by $\varepsilon2/\varepsilon2$ allele has a low binding force to the receptor and is considered to cause hyperlipidemia.

In this Experimental Example, first, astrocytes expressing apolipoprotein of each isoform were induced to differentiate in the same manner as in Experimental Example 1 except that iPS cells having APOE loci of ε2/ε2 allele, ε3/ε3 allele, and ε4/ε4 allele were used.

Subsequently, each of the obtained astrocytes was co-cultured with neurons. As the neurons, neurons induced to differentiate from iPS cells were used. Further, neurons not co-cultured with astrocytes were used as controls.

First, an expression plasmid for fusion protein (GFP-Actin) of green fluorescent protein (GFP) and actin was introduced into neurons using the liposome method. Subsequently, co-culture with astrocytes was started within 2 weeks after the introduction of the expression plasmid. Subsequently, within 7 days from the start of co-culture, cells of each co-culture system were paraformaldehyde-fixed, and nerve spines were detected.

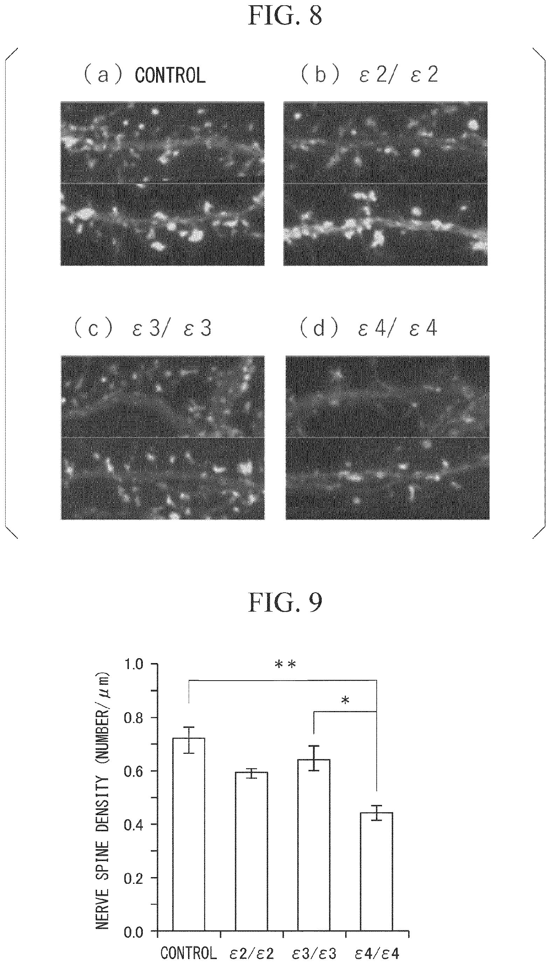

Figs. 8A to 8D are fluorescence micrographs showing the results of detecting the fluorescence of GFP. The observation results are shown for two fields of view each. Fig. 8A shows the results of neurons not co-cultured with astrocytes, Fig. 8B shows the results of neurons co-cultured with astrocytes having ε2/ε2 allele, Fig. 8C shows the result of neurons co-cultured with astrocytes having ε3/ε3 allele, and Fig. 8D shows the result of the neuron co-cultured with astrocytes having ε4/ε4 allele.

Further, Fig. 9 is a graph showing the nerve spine density measured based on the results of Figs. 8A to 8D. In Fig. 9, "*" indicates that there is a significant difference at $p < 0.05$ as a result of one-way ANOVA based on Tukey's post-hoc test, and "**" indicates that there is a significant difference at $p < 0.01$.

As a result, it was revealed that the nerve spine density was significantly reduced in the neurons co-cultured with astrocytes having ε4/ε4 allele.

[Experimental Example 7]

(Functional analysis 2 of astrocytes induced to differentiate from human iPS cells)

A system for evaluating astrocyte activity was constructed. First, human iPS cells were induced to differentiate in the same manner as in Experimental Example 1 to produce astrocytes. As human iPS cells, iPS cell lines 201B7, WD39, and 1210C1 derived from a healthy person, and an iPS cell line AD derived from an Alzheimer's disease patient were used.

Subsequently, in the same manner as in Experimental Example 1, each astrocyte passaged up to A3 or later was seeded on a 96-well plate at a cell density of $5 \times 10^4$ to $1 \times 10^5$ cells/plate. Subsequently, each astrocyte was immobilized and fluorescently immunostained with astrocyte activity marker GFAP using an anti-GFAP antibody. Subsequently, the fluorescence intensity of each astrocyte was measured at the level of one cell using an imaging analyzer (product name "In Cell analyzer", GE Healthcare).

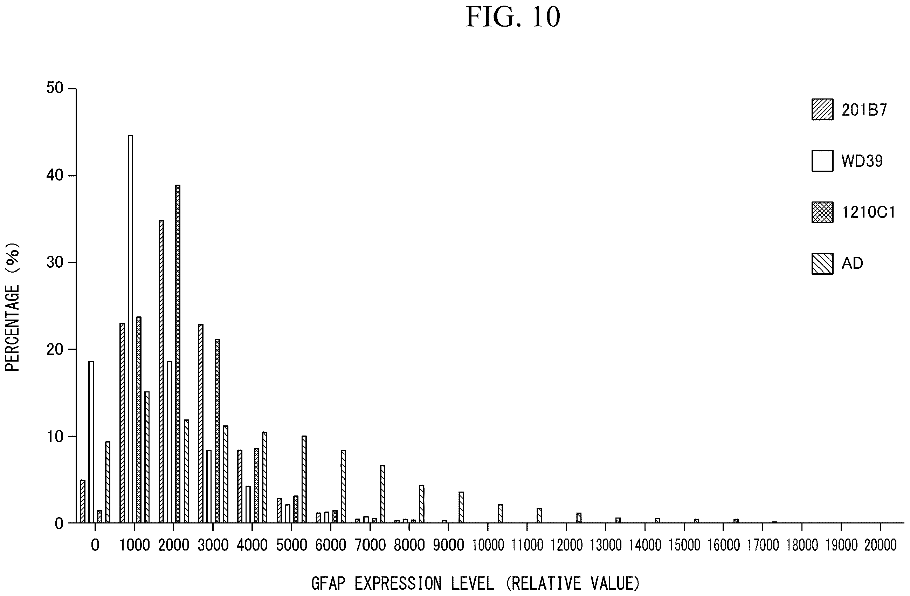

Fig. 10 is a histogram of fluorescence intensity (relative value) measured for each astrocyte. As a result, it was found that the percentage of cells having a low GFAP expression level was high in astrocytes induced to differentiate from iPS cells derived from a healthy person, whereas the percentage of cells having a higher GFAP expression level increased in astrocytes induced to differentiate from iPS cells derived from an Alzheimer's disease patient, and the peak of the histogram tended to move to the right.

This result indicates that astrocytes induced to differentiate from iPS cells derived from an Alzheimer's disease patient have increased activity. The result further indicates that astrocytes induced to differentiate from iPS cells derived from an Alzheimer's disease patient reflect the pathophysiology of Alzheimer's disease, and that they can be used as a model for Alzheimer's disease.

Industrial Applicability

According to the present invention, it is possible to provide a technique for producing a cell population of mature astrocytes in a static state.

Claims

1. A method for producing astrocytes, comprising:

   dissociating an embryoid body into single cells and suspension-culturing the cells in a serum-free medium containing basic fibroblast growth factor (bFGF) and epidermal growth factor (EGF) to obtain a neural stem cell mass; and
   dissociating the neural stem cell mass into single cells and adhesion-culturing the cells in a serum-free medium to obtain a cell population containing astrocytes.

2. The method for producing astrocytes according to Claim 1,
   wherein the embryoid body is obtained by culturing pluripotent stem cells in a serum-free medium.

3. The method for producing astrocytes according to Claim 2,
   wherein the pluripotent stem cells are induced pluripotent stem cells derived from a healthy person or induced pluripotent stem cells derived from a neurological disease patient.

4. The method for producing astrocytes according to any one of Claims 1 to 3,
   wherein obtaining the neural stem cell mass includes:

   dissociating the embryoid body into single cells and suspension-culturing the cells in a serum-free medium containing bFGF and EGF to obtain a primary neural stem cell mass; and
   dissociating the primary neural stem cell mass into single cells and suspension-culturing the cells in a serum-free medium containing bFGF and EGF to obtain a higher-order neural stem cell mass.

5. The method for producing astrocytes according to any one of Claims 1 to 4,
   wherein in obtaining the cell population containing the astrocytes, the serum-free medium further contains brain-derived neurotrophic factor (BDNF) and/or glial-cell derived neurotrophic factor (GDNF).

6. The method for producing astrocytes according to any one of Claims 1 to 5,
   wherein a percentage of the astrocytes in the cell population is 90% or more.

7. The method for producing astrocytes according to any one of Claims 1 to 6,
   wherein the astrocytes are in a static state.

8. A cell population contained in a container in a serum-free state, wherein a percentage of astrocytes is 90% or more.

9. The cell population according to Claim 8, which is in a static state.

10. The cell population according to Claim 8 or 9, wherein a cell density is $1 \times 10^5$ cells/mL or more.

11. The cell population according to any one of Claims 8 to 10,
    wherein the cell population includes 95% or more of glial fibrillary acidic protein (GFAP)-positive cells and 95% or more of S100 calcium binding protein B (S100B)-positive cells.

12. The cell population according to any one of Claims 8 to 11, wherein when serum is added to the medium, an expression level of GFAP, NFIA, ALDH1L1 or EAAT2 increases.

13. A co-culture of the cell population according to any one of Claims 8 to 12 and neurons.

14. A method for screening a therapeutic agent for a neurological disease, comprising:

    co-culturing the cell population according to any one of Claims 8 to 12 and neurons in the presence of a test substance; and
    evaluating neurite outgrowth, morphology, synaptic vesicles, gene expression, protein expression, or electrophysiological parameters of the neurons to obtain an evaluation result,
    wherein a significant change in the evaluation result as compared to an evaluation result in the absence of the test substance indicates that the test substance is a candidate for a therapeutic agent for neurological diseases.

15. A serum-free medium for inducing differentiation of neural stem cells into astrocytes, comprising transferrin, putrescine, insulin, progesterone, sodium selenite, and a B27 supplement.

16. The serum-free medium for inducing differentiation of neural stem cells into astrocytes according to Claim 15, further comprising BDNF and/or GDNF.

FIG. 1

Neural Stem Cells (NS)

| iPS cells | Embryoid body | Primary NS | Secondary NS | Neurons and Astrocytes 30 days | Astrocytes |
|---|---|---|---|---|---|

16 days → 16 days → 16 days → 3 times passage →

| DMEM/F12 | | MHM/B-27 supplement | | | |
|---|---|---|---|---|---|
| 20% KSR | 5% KSR | | | | |
| | 3i | 1μM RA, 1μM PM | 20ng/ml bFGF, 10ng/ml EGF 1μM PM | 20ng/ml bFGF, 10ng/ml EGF | 10ng/ml BDNF, 10ng/ml GDNF |

# FIG. 2

FIG. 3

EP 3 851 521 A1

# FIG. 4

FIG. 5

# FIG. 6

## FIG. 7

# FIG. 8

(a) CONTROL

(b) ε2/ε2

(c) ε3/ε3

(d) ε4/ε4

# FIG. 9

FIG. 10

EP 3 851 521 A1

## INTERNATIONAL SEARCH REPORT

| International application No. |
| --- |
| PCT/JP2019/035318 |

**A. CLASSIFICATION OF SUBJECT MATTER**
Int.Cl. C12N5/0797(2010.01)i, C12N5/02(2006.01)i, C12Q1/06(2006.01)i

According to International Patent Classification (IPC) or to both national classification and IPC

**B. FIELDS SEARCHED**

Minimum documentation searched (classification system followed by classification symbols)
Int.Cl. C12N5/0797, C12N5/02, C12Q1/06

Documentation searched other than minimum documentation to the extent that such documents are included in the fields searched
Published examined utility model applications of Japan     1922–1996
Published unexamined utility model applications of Japan   1971–2019
Registered utility model specifications of Japan           1996–2019
Published registered utility model applications of Japan   1994–2019

Electronic data base consulted during the international search (name of data base and, where practicable, search terms used)
JSTPlus/JMEDPlus/JST7580 (JDreamIII),
CAplus/WPIDS/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y | JP 2016-535248 A (KADIMASTEM LTD.) 10 November 2016, claims, paragraphs [0029], [0089]–[0097], [0156]–[0159] & US 2016/0237404 A1, claims, examples & WO 2015/049677 A1 & EP 3052191 A1 & KR 10-2016-0058822 A & CN 105960453 A | 8-2,14-16<br>1-16 |
| X<br>Y | JP 2016-504014 A (KYOTO UNIVERSITY) 12 February 2016, claims, paragraphs [0021], [0029], [0038], [0069], [0072]–[0077], fig. 1-5 & US 2015/0353888 A1, claims, examples, fig. 1-5 & WO 2014/104409 A1 & EP 2938721 A1 | 8-14<br>1-16 |

☒ Further documents are listed in the continuation of Box C.     ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 15 November 2019 (15.11.2019) | 26 November 2019 (26.11.2019) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
| --- | --- |

Form PCT/ISA/210 (second sheet) (January 2015)

**INTERNATIONAL SEARCH REPORT**

| International application No. |
| --- |
| PCT/JP2019/035318 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | 松本有史 ほか，神経幹細胞の分化制御機構，ファルマシア，2003, vol. 39, no. 2, pp. 108-112, non-official translation (MATSUMOTO, Arifumi et al., "Differentiation control mechanism of neural stem cell", Farumashia) | 1-16 |

Form PCT/ISA/210 (continuation of second sheet) (January 2015)

**REFERENCES CITED IN THE DESCRIPTION**

**Patent documents cited in the description**

- JP 2018172407 A

- JP 2016504014 W